# EUROPEAN PATENT SPECIFICATION

(11) **EP 4 282 341 B1**
(45) Date of publication and mention of the grant of the patent: **19.11.2025**
(21) Application number: 22185144.7
(22) Date of filing: 15.07.2022
(51) Int. Cl.: A61B 10/00, A61B 10/02

(54) **SAMPLE COLLECTOR**
PROBENSAMMLER
COLLECTEUR D'ÉCHANTILLON

(30) Priority: 27.05.2022 CN 202210594528; 09.06.2022 US 202263350572 P
(43) Date of publication of application: 29.11.2023
(73) Proprietor: Hangzhou Biotest Biotech Co., Ltd., Yuhang District Hangzhou (CN); Advin Biotech, Inc., San Diego, CA 92121 (US)
(72) Inventor: WANG, Bailong, Hangzhou (CN); WU, Shujiang, Hangzhou (CN); WU, Junsheng, Hangzhou (CN); LI, Shanying, Hangzhou (CN)
(74) Representative: Piotrowicz, Pawel Jan Andrzej

(56) References cited:
- EP-A1- 0 412 610
- WO-A1-2021/195633
- WO-A1-2021/217025
- CN-U- 201 993 241
- US-A1- 2015 157 251
- US-A1- 2017 143 314
- US-A1- 2021 330 302

## Description

The present invention claims the priority of the prior Chinese application with the application number of 202210594528.0 applied on May 27, 2022, and the prior US application with the application number of 63/350,572 applied on June 9, 2022.

### TECHNICAL FIELD

The present invention relates to the technical field of in-vitro diagnosis, in particular, relates to an innovative application of a melamine foam in in-vitro diagnosis, and in particular to an application of a melamine foam in sample collection or processing.

### BACKGROUND

Sample collection or processing is a key step in the in-vitro diagnostic process, and is directly related to the accuracy and sensitivity of diagnostic results. Existing sample collection or processing usually uses a sponge head for sample processing, or uses a filter membrane for sample filtration, but because the sponge head or the filter membrane has an adsorption effect on a target determinand in a sample, especially on low-content or easy-to-adsorb detection markers, target detection substances are difficult to detect, resulting in false detection or missing detection, and false negative or false positive diagnostic results. Therefore, it is urgent to find a device that can significantly reduce adsorption of the target determinand during sample collection and processing, or can significantly improve an elution effect of the target determinand to restore authenticity of samples and improve sensitivity of product detection.

D1 (US 2017/143314 A1) discloses a biological fluid sampling vessel including a container portion having an opening and an interior for housing a urine sample therein. The vessel may further include a sampling portion that removably engages the container portion to close off the interior. The sampling portion may include a sampling wand configured for obtaining a sample.

The sampling wand may comprise melamine foam.

WO 2021/195633 A1 discloses a device for collecting and subsequently analysing a liquid sample, inter alia for THC compounds or metabolites.

### SUMMARY

According to an aspect of the present invention, there is provided a detection device according to claim 1 and a detection method according to claim 13. Optional features of the device are outlined in claims 2 to 12, and optional features of the method are outlined in claims 14 to 15.

### BRIEF DESCRIPTION OF DRAWINGS

FIG. 1 is a structural top view of a test strip in a specific embodiment.
FIG. 2 is a schematic diagram of a three-dimensional structure of a test strip in a specific embodiment.
FIG. 3 is a colourimetric card (standard) for drug abuse detection of the present invention.
FIG. 4 shows fluorescent reading values (X1/X2) on a detection area on a test strip after standards with THC of 20 ng/mL are absorbed by different materials and squeezed (Embodiment 2.3) (No. 1 to 3 are melamine foams, and No. 4 to 6 are polypropylene).
FIG. 5A is a picture of detection results and control test results of samples released by squeezing after urine standards of 20 ng/mL are absorbed by different materials.
FIG. 5B is a picture of detection results and control test results of samples released by squeezing after urine standards of 30 ng/mL are absorbed by different materials.
FIG. 6A is a peak value graph of a standard solution of 100 ng/ml tetrahydrocannabinol Δ9-THC (an injection volume of 10 µl).
FIG. 6B is a peak value graph of HLPC with an injection volume of 10 µl of samples after 500 µl of 100 ng/ml Δ9-THC is absorbed by a sampling head prepared from a melamine foam and is squeezed and released.
FIG. 6C is a peak value graph of HLPC with an injection volume of 10 µl of samples after 500 µl of 100 ng/ml Δ9-THC is absorbed by a sponge head (polypropylene sponge) prepared from polypropylene and is squeezed and released.
FIG. 7 is an infrared light scanning analysis chromatogram of a melamine foam.
FIG. 8 is an infrared light scanning analysis chromatogram of a polypropylene sponge.

### Detailed Description

The structures involved in the present invention or the technical terms used are further described below. If there is no special indication, they should be understood and interpreted according to the general terms commonly used in the art.

### Detection

Detection means assaying or testing for the presence or the absence of a substance or material such as, but not limited to, chemicals, organic compounds, inorganic compounds, metabolites, drugs or drug metabolites, organic tissue or metabolites thereof, nucleic acids, proteins or polymers. In addition, detection means testing the quantity of substances or materials. Further, assaying also means immunodetection, chemical detection, enzymatic detection, gas chromatography, mass spectrometry, nucleic acid amplification and the like. Any detection tool, any method, and any device that can detect analytes in samples can be used in the present invention to detect the analytes in the samples. It is only that these samples are processed by melamine foams or melamine materials of the present invention and filtered for detection.

### Samples or specimens

Samples collected or processed by melamine foams of the present invention include biological fluids (e.g., case fluids or clinical samples). Samples or specimens here are interchangeable and can mean the same thing. Liquid samples or fluid samples can be derived from solid or semi-solid samples, including excreta, biological tissue and food samples. The solid or semi-solid samples can be converted to a liquid samples by any suitable method, such as mixing, mashing, macerating, incubating, dissolving or using enzymolysis in a suitable solution (e.g., water, a phosphate solution or other buffer solutions) to digest solid samples. "Biological samples" include samples derived from animals, plants and food, such as urine, saliva, blood and components thereof, spinal fluid, vaginal secretions, sperms, feces, sweat, secretions, tissue, organs, tumors, tissue and organ cultures, cell cultures and media derived from human or animals. A preferred biological sample is urine, preferably a biological sample is saliva. Food samples include food processing substances, final products, meat, cheese, wine, milk and drinking water. Plant samples include plant tissue, plant cell cultures and media derived from any plant. "Environmental samples" are derived from the environment (e.g., liquid samples derived from lakes or other water bodies, sewage samples, soil samples, groundwater, seawater, and waste liquid samples). The environmental samples may also include sewage or other waste water.

By using a suitable melamine foam of the present invention, any sample can be collected and processed to reduce adsorption of analytes. Preferably, small drug molecules in the saliva and the urine are collected by using the melamine foam of the present invention. Of course, the samples collected or processed by using the melamine foam of the present invention may be samples in any of the above forms, whether initially solid or liquid, as long as these liquids or liquid samples can be absorbed by the melamine foam.

When a test element is used for detection, the test element generally has a sample application area. The sample application area here is generally prepared from a water-absorbing material, and liquid samples or fluid samples can be absorbed through the capillary or other properties of a material of an absorbing element, so that the fluid samples flow in the sample application area. A material of the sample application area for the fluid samples may be any material that can absorb liquids, such as the melamine foam of the present invention, or a sponge and filter paper mixed with the melamine foam, polyester fiber, gel, non-woven fabrics, cotton, polyester films, yarns, etc. The melamine foam can be mixed in the above non-melamine foam water-absorbing materials, so that the adsorption effect of these water-absorbing materials on the analytes in the samples can also be reduced. When detection is needed, a buffer solution is applied to the sample application area to dissolve the samples, so that the dissolved samples flow on the test element or a detection element.

### Downstream and upstream

Downstream or upstream is divided for a liquid flow direction, generally a liquid or fluid flows from an upstream area to a downstream area. The downstream area receives a liquid from the upstream area, and the liquid can also flow along the upstream area to the downstream area. This is generally divided according to the liquid flow direction. For example, on some materials that use capillary force to promote liquid flow, the liquid can overcome gravity and flow in a direction opposite to the gravity. At this time, the upstream and downstream are still divided according to the liquid flow direction. For example, in a detection device of the present invention, after a test element absorbs a fluid sample or a liquid sample, a fluid can flow from a sample application area 101 of the test element to a detection area 103 of the test element 1, and at this time, the flow of the liquid from the sample application area 101 to the detection area 103 is from upstream to downstream. In the process of circulation, it passes through a test area 102, and there is a detection area 105 and a test result control area 106 on the test area. The test area may be a polyester fiber film, and the sample application area may be glass fiber. At this point, the sample application area or absorption area 101 is located upstream of the sample application area of the test element.

### Test elements

So-called "test elements" here refer to elements that can detect whether samples or specimens contain interested analytes. This detection is based on whatever technical principles, such as immunology, chemistry, electricity, optics, molecules, nucleic acids, physics, etc. After the samples are collected or processed by using a melamine foam provided by the present invention, the samples that pass through the melamine foam can be directly applied onto the test elements for testing or assaying.

The test elements here may be selected from a lateral flow test strip, which can detect a variety of analytes. Of course, other suitable test elements may also be applied in the present invention. Various test elements may be combined to be applied in the present invention. One form is test paper. Test paper for analysis of analytes (such as drugs or metabolites indicative of medical conditions) in a sample may be in various forms, such as forms of immunoassay or chemical analysis. The test paper may use a non-competitive or competitive analysis mode. The test paper generally includes a water absorption material with a sample adding area, a reagent area and a test area. A fluid or liquid sample is added to the sample adding area and flows to the reagent area by capillary action. In the reagent area, the sample binds to a reagent if the analytes are present. The sample then continues to flow to the test area. For other reagents, if molecules that specifically bind to the analytes are immobilized in the test area, these reagents react with the analytes (if present) in the sample and bind the analytes in this area, or bind to a certain reagent in the reagent area. A label used for displaying a detection signal is present in the reagent area or a separate labeling area.

A typical non-competitive analysis mode refers to that a signal will be generated if the sample contains the analytes, and no signal will be generated if the sample does not contain the analytes. If the greater the number of the analytes, whose concentration exceeds a threshold, in the sample, the stronger the signal. In a competition method, a signal will be generated if the analytes are not present in the sample, and no signal will be generated if the analytes are present. Likewise, if the greater the number of the analytes, whose quantity exceeds a threshold, in the sample, the weaker the signal produced in a signal region where they are. The "threshold" here is a clinical or general value, and can be adjusted according to different situations. For example, when the competition method is used for detecting the analytes, gold particles (black or purple) are used as markers for signal generation. Generally, when the concentration in the sample exceeds the threshold, it is hoped that no signal will be generated, indicating a positive result. When the concentration in the sample is less than the threshold, it is hoped that a signal will be generated, indicating a negative result. The form of the signal may be a line, a circular point, or any other form. When there is a color line, a standard color card is usually used for comparing the concentration of the color or the depth of the color to judge the standard of a negative result or a positive result. Usually, the less the analytes in the sample, the darker the color line. On the contrary, the more the content of the analytes in the sample, the lighter the color line, thereby indicating the quantitative level or concentration of the content of the analytes in the sample.

The test elements may be test paper, which may be selected from materials that absorb or do not absorb water. The test paper may include a variety of materials for liquid sample transfer. One of the materials of the test paper can cover another material, for example, filter paper covers a nitrocellulose membrane. One of the areas of the test paper may be selected from one or more materials, while the other area is selected from other different one or more materials. The test paper can be adhered to certain support or hard surface to improve the strength of holding the test paper.

The analytes are detected by a signal generating system, for example, by using one or more enzymes that specifically react with the analytes, and by using the method of immobilizing the specific binding substance on the detection test paper as described above, one or more combinations of the signal generating system are immobilized on the analyte test area of the test paper. A substance generating signals may be in the sample adding area, the reagent area, or the test area, or on the entire test paper, and the substance may fill on one or more materials of the test paper. A signal-substance-containing solution is added to the surface of the test paper or one or more materials of the test paper are immersed in the signal-substance-containing solution. The test paper to which the signal-substance-containing solution is added is dried. The signal here may be any signal system, such as colored particles such as gold particles, latex particles or colored dyes, and of course also fluorescent substances.

The respective areas of the test paper may be arranged in the following ways: a sample adding area 101, a reagent area, a test area 103, a control area, an area for determination of whether a sample is adulterated or not, and a liquid sample absorption area. The control area is located behind the test area. All the areas may be arranged on a strip of test paper using only one material. Different materials may also be used for different areas. The respective areas may be in direct contact with the liquid sample, or different areas are arranged according to the flow direction of the liquid sample, and a tail end of each area is connected and overlapped with a front end of another area. The materials used may be materials with a better water absorption property, such as filter paper, glass fiber or nitrocellulose membranes. The test paper may also use other forms.

Generally, a commonly used reagent strip is a nitrocellulose membrane reagent strip, that is, a detection area includes a nitrocellulose membrane (NC), and specific binding molecules are immobilized on the nitrocellulose membrane to display detection results; it may also be a cellulose acetate membrane or a nylon membrane, etc. For example, reagent strips or devices containing reagent strips are described by the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620; and US 6403383. The test strips and similar devices with test strips disclosed in the above patent documents can all be applied to the test elements or detection devices of the present invention to detect the analytes, such as the detection of the analytes in the sample.

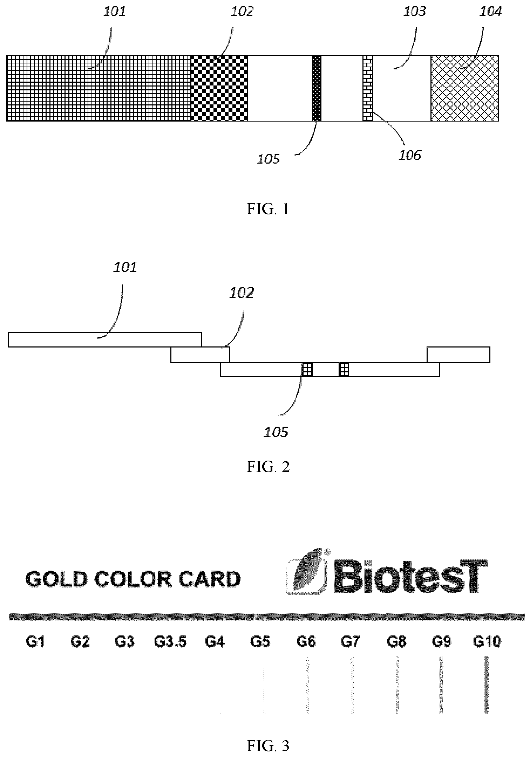

Detection reagent strips applied to the present invention may be commonly called lateral flow test strips. The specific structures and detection principles of these detection reagent strips are technologies well known to those of ordinary skill in the art in the prior art. A common detection reagent strip (FIG. 1) includes a sample collection area or a sample adding area 101, a labeling area (not shown), a detection area 103 and a water absorption area. The sample collection area includes a sample receiving pad. The labeling area includes a labeling pad. The water absorption area may include a water absorption pad. The detection area includes necessary chemical substances, such as immunological reagents or enzymatic chemical reagents, which can detect whether an analyte is contained or not. Generally, a commonly used detection reagent strip is a nitrocellulose membrane reagent strip, that is, a detection area 103 includes a nitrocellulose membrane, and it is an area for displaying detection results by immobilizing specific binding molecules on the nitrocellulose membrane; it may also be a cellulose acetate membrane or a nylon membrane, etc. Of course, a detection result control area may also be included downstream of the detection area. Usually, the control area and the detection area appear in a form of horizontal lines, which are detection lines or control lines. Such detection reagent strips are traditional reagent strips, and of course, they may also be other types of reagent strips that utilize capillary action for detection. In addition, general detection reagent strips have dry chemical reagent components, such as immobilized antibodies or other reagents. When they encounter a liquid, the liquid flows along the reagent strips with capillary action, and with the flow, the dry reagent components are dissolved in the liquid, thus proceeding to the next area, the dry reagents in this area are processed, to perform necessary detection. Liquid flow proceeds mainly through capillary action. Here, all of them can be applied to the detection device of the present invention, or arranged in a detection chamber to be in contact with a liquid sample, or used for detecting the presence or the absence of an analyte in the liquid sample entering the detection chamber or the quantity of the presence thereof.

In addition to that the above test strips or the lateral flow test strips themselves are used for being in contact with the liquid sample to test whether the liquid sample contains the analyte, the test element of the present invention itself can be used as a detection device to detect the analyte in the sample, so the detection device itself is equivalent to the test element here. For example, after a fluid sample is mixed with a treatment liquid, the test element is directly used for detection. As will be described in detail below, the test element may be used alone for detection while it is described that a receiving device is used for processing the fluid sample.

### Analytes

Examples that can use analytes involved in the present invention include some small molecular substances, including drugs (e.g., drugs of abuse). "Drugs of abuse" (DOA) refers to the use of drugs for non-medical purposes (usually playing a role in paralyzing nerves). Abuse of these drugs will lead to physical and mental damage to cause dependence, addiction and/or death. Examples of drugs of abuse include cocaine; amphetamine AMP (e.g., Black Beauty, white amphetamine tablets, dextroamphetamine, dexedrine tablets, Beans); methamphetamine MET (crank, methamphetamine, crystal, speed); barbiturate BAR (such as Valium , Roche Pharmaceuticals, Nutley, New Jersey); sedatives (i.e., sleep aid drugs); lysergic acid diethylamide (LSD); inhibitors (downers, goofballs, barbs, blue devils, yellow jackets, methaqualone); tricyclic antidepressants (TCA, i.e., imipramine, amitriptyline, and doxepin); methylene dioxymetham-phetamine MDMA; phencyclidine (PCP); tetrahydrocannabinol (THC, pot, dope, hash, weed, etc.); opiates (i.e., morphine MOP or opium, cocaine COC; heroin, oxycodone); anxiolytics and sedative-hypnotics, and the anxiolytics are a class of drugs mainly used for reducing anxiety, tension and fear and stabilizing mood and having both hypnotic and sedative effects, including benzodiazepines BZO, atypical BZs, fusion diazonium NB23Cs, benzodiazepines, ligands for BZ receptors, and open-ring BZs, diphenylmethane derivatives, piperazine carboxylates, piperidine carboxylates, quinazolinones, thiazine and thiazole derivatives, other heterocycles, imidazole-type sedatives/analgesics (such as oxycodone OXY, methadone MTD), propylene glycol derivatives - carbamates, aliphatic compounds, anthracene derivatives, etc. A detection device using the present invention may also be used for detection of drugs that belong to medical purposes but are prone to overdose, such as tricyclic antidepressants (imipramine or analogues) and acetaminophen. These drugs will be metabolized into small molecular substances after being absorbed by the human body. These small molecular substances exist in blood, urine, saliva, sweat and other body fluids or in some body fluids.

For example, analytes detected with the present invention include, but are not limited to, creatinine, bilirubin, nitrite, proteins (non-specific), hormones (e.g., human chorionic gonadotropin, progesterone hormone, follicle stimulating hormone, etc.), blood, leukocytes, sugars, heavy metals or toxins, bacterial substances (such as proteins or carbohydrates against specific bacteria, e.g., *Escherichia coli* 0157:H7, *Staphylococcus, Salmonella, Clostridium, Campylobacter, L. monocytogenes, Vibrios,* or *Bacillus cereus*) and substances associated with physiological characteristics in urine samples, such as pH and proportion. Any other clinical urine chemical analysis can use a lateral flow detection form to cooperate with the device of the present invention for detection.

### Melamine material

A main component of the melamine material described here is melamine resin or melamine and phenolic resin, which is a copolycondensation product mainly composed of melamine, phenol and formaldehyde. A material used in the present invention for processing or collecting samples is a material made by polymerization of melamine as a main raw material, or a material directly synthesized by using formaldehyde and melamine as raw materials. In some embodiments, the material of the present invention includes a porous material produced by a foaming process after formaldehyde and melamine are directly synthesized as raw materials. The "foaming" here can be used for making materials containing micropores or multiple pores by any existing method. The foaming process is also in the prior art, for example, foaming resin, a foaming assistant and binder resin (making a finished product adhesive) are mixed together; foam processing is conducted as follows: 80 parts of ethylene vinyl acetate (EVA), 20 parts of APAO PT 3385, 20 parts of azodicarbonamide, 19 parts of CaCO and 0.6 part of dicumyl peroxide are mixed together and placed in a mold for foaming, and closed pores are broken by mechanical force, so that a foamed sponge can be obtained. Its density (d) is generally 0.028 g/cm, and the 25% compressive hardness is 1.9 KPa. Just like foaming, the same material and different manufacturing processes will make different things. A process of how to carry out foaming may also be performed by referring to the scheme described in the Chinese invention patent, patent publication CN110774604B, and any technology in the specification of the patent can be used as a specific implementation of the present invention to prepare a foamed sponge. A production process of a foaming material may also be performed by referring to any technical solution in the Chinese Invention Patent Application Publication, Publication No. CN107553920A, which specifically discloses a pore opening method for a foamed sponge, and a foaming material produced by the method disclosed in this patent application may also be used for a melamine material of the present invention for foaming treatment. It can be understood that the foaming process is only a process for producing a porous material, and of course any other method can also be used for producing the porous material.

Of course, in some embodiments, the melamine material or melamine porous material, also known as a melamine foam material, of the present invention, can be commercially purchased, and many existing commercial companies specialize in producing this material. Customized processing can also be carried out, a production company is told a ratio of formaldehyde to melamine, or a required porous size, the number of pores under unit volume, etc. In this way, required porous materials can be produced.

In some embodiments, the melamine material here is a porous water-absorbing material. The "water-absorbing" here means that a liquid can be absorbed while it is retained in the material. A sample is generally absorbed by capillary action. In some embodiments, after a porous material absorbs a liquid, the liquid can also be released from the porous material. Releasing here means that the porous material can be squeezed physically, to allow the liquid to flow out of the porous material. Alternatively, some liquids are used for soaking the porous material, so that a liquid sample absorbed by the material is dissolved in a processing liquid. The processing liquid here is not a liquid sample, but a reagent used for dissolving the sample, such as some pH adjustment reagents, buffer solutions, and lysis solutions. There are also many squeezing ways, all of which are to reduce a volume of the porous material at some point, so that the absorbed liquid sample is released from the porous material, and this is because the porous material is compressed and the liquid flows out of it.

In existing traditional industry, the so-called melamine foam, also known as a melamine foam or a melamine-melamine foam, is a novel sound-absorbing, heat-insulating and heat-preservation materials successfully developed in the 1990s internationally. In addition to its excellent sound absorption, heat insulation and fire resistance, the melamine foam has the advantages of high flame retardancy, environmental protection, hygienic property and safety compared with similar products such as glass wool and polyurethane foam.

However, the inventors of the present invention found that when this special material is used for absorbing and processing a sample, adsorption of an analyte can be reduced. In particular, the adsorption of the following substances in the sample can be well improved, such as tetrahydrocannabinol (THC). In existing drug abuse tests, a problem of adsorption of tetrahydrocannabinol is a difficult problem, which is that a material of a traditional collector has an adsorption effect on THC (tetrahydrocannabinol) in the sample, resulting in missing detection or false negative results. For example, when a concentration of THC in the sample is A, a content of THC in a sample collected by the traditional collector and squeezed from the collector is less than A or much less than A, a test value obtained in this way is in error with a real value in an actual sample, and this error may cause missing detection. Especially in some products using in-vitro diagnostic reagents, a sample is collected generally by using a collector and then squeezed from the collector, and then a squeezed sample is tested. If a material used for absorbing a sample only as a collector has an adsorption effect, missing detection or false negative results will be often caused. Of course, sometimes it is not necessary to use a collector to collect a sample, and sometimes it is necessary to pre-process the sample before testing, such as filtering, dissolving or eluting impurities in the sample before testing. Since a filtering material used has adsorption to an analyte, it will also cause errors between test results and actual results. After the melamine material of the present invention is used for sample collection and processing, it is found that the analyte, especially THC, is rarely adsorbed, so that an obtained detection value is close to a real value of the sample itself, avoiding missing detection or false negative wrong results.

In some embodiments, the tetrahydrocannabinol here generally includes Δ9-THC (Δ9-tetrahydrocannabinol), a molecular formula thereof being C₂₁H₃₀O₂, and a chemical structure thereof being as follows, or includes some of similar substances thereof:

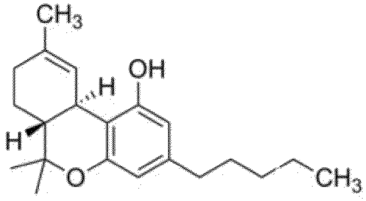

In some another embodiments, the tetrahydrocannabinol here generally includes 11-desmethyl-Δ9-tetrahy drocannabinol-9-carboxylic acid (11-nor-Δ9-THC-9COOHΔ9-tetrahydrocannabinol), a molecular formula thereof being C₂₁H₂₈O₄, a chemical structure thereof being as follows, or includes similar substances similar to it:

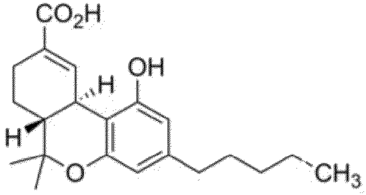

The similar substances here are mainly that its main structure is similar to tetrahydrocannabinol, but groups in respective chemical space structures have different substituents and different chemical substances appear, but their main structures are the same or similar to tetrahydrocannabinol.

**In** some embodiments, the melamine material used in the present invention is used for collecting or processing a sample, so that an adsorption effect on an analyte can be reduced. "Collecting" here refers to that a collector prepared or made with a melamine-containing material is used for absorbing a sample or absorbing a fluid sample, which can be squeezed directly from an absorber after absorption, or a liquid sample eluted from the absorber. This collecting may be for absorbing a sample, or for absorbing a sample from a human body or an organism, such as collecting saliva and sputum samples from an oral cavity of a human or mammal, or sucking some samples from human urine and fecal samples for subsequent detection. Of course, it may also be that the melamine material of the present invention is used for directly collecting a solid sample or a semi-solid sample, which are then dried and then stored or transported, and when detection is required, the sample on a collector is dissolved by a liquid and becomes a liquid sample in form. **In** some embodiments, a porous material containing a melamine material can be used for absorbing a liquid or fluid sample, and is then stored or transported to a detection site, the fluid sample is squeezed from the porous material before detection, and the fluid sample is then detected. **In** some embodiments, all the entire portion for absorbing a sample of a collector consists of a melamine material or a melamine material porous material (melamine foam). Of course, the portion for absorbing the sample may mainly consist of the melamine material or the melamine material porous material (melamine foam), and also contain other non-melamine materials, for example, the melamine material accounts for 55%, 60%, 65%, 70%, 75%, 80%, 85%, 90%, 95% and 99% of a total absorbing material.

The "adsorption" here mainly refers to adsorption of an analyte in a sample by a collecting material when a liquid sample is collected or processed. This adsorption may be physical adsorption or adsorption by chemical bonds on a material acting with chemical groups of the analyte, thus elution again from the collecting material is relatively difficult by conventional methods. The present invention has unexpectedly found that when a melamine-containing material or a melamine porous material is used for collecting a sample, an analyte is less adsorbed and a large part or most of the analyte can be eluted, or after an absorbing material is squeezed, most of the analyte can flow out of the absorbing material with a liquid sample, and a small part of the analyte remains in the absorbing material. It is not clear what causes the use of the melamine material to collect or process the sample to reduce "adsorption", possibly a dual effect of physics or chemistry.

It needs be specially noted here that the melamine material may be a material without pores or a material with pores. **In** some preferred embodiments, it is a water-absorbing material with pores, and here the pores can be produced generally by foaming techniques. Of course, the melamine material can also be used for making line-like and fibrous hair styles. Each fiber of these fibers or hairs does not have a pore structure, but when multiple fibers are gathered together, there are gaps or pores between the fibers, such as micropores and tiny gaps. The sample can also be absorbed or processed by capillary action. For example, it is also possible to use fibers made of the melamine material to make flocking cotton swabs. Methods for making flocking cotton swabs are specifically disclosed in the prior art, for example, referring to the following patents: US8,114,027, US8,317,728, US 8,979,784, US 9,011,358, US 9,173,779, US 10,327,741, AU2004226798, JP4579902, ZL200610099310.9 for specific production. Therefore, the so-called melamine material here may be a porous material or a material with pores. There are no fixed requirements for the size of the pores. Generally, pores with capillary action are also acceptable, or porous materials that absorb liquid samples are also acceptable. Of course, it can be used for making an absorbing material on a test element. Therefore, in addition to directly using the porous melamine material to absorb or process the sample, other "water-absorbing" devices made of the melamine material can also be used for absorbing or processing the sample, for example, a sample application area 101 of the test element may be a fibrous pad made of the melamine material for sample application or reception, so that a fluid can be allowed to flow on a sample pad.

The "processing" here refers to processing of a sample with a melamine-containing material or a melamine porous material before detection. The processing here may include the meaning of collection, or may include allowing a liquid sample to pass or flow through the melamine-containing material or the melamine porous material, so in order to achieve the processing of the sample before the detection, it is hoped that an analyte will not be damaged, such as filtering to remove impurities, for example, some other impurities that are not target substances in saliva need to be filtered out, such as proteins or enzymes, or filtering blood to remove red blood cells, etc. Therefore, in some embodiments, a melamine-containing material or a melamine porous material can be used as a collector, and can also be used for making a part of a test strip, for example, as a material for a sample application area of the test strip, or as a material for a labeling area, or a material of a detection area. After all, the melamine porous materials can be produced in a customized mode, and having a porous material can allow for capillarity flow of fluid samples. The porous material here may be multiple pores with capillary action, or a porous material formed by a "foaming" process.

### DETAILED DESCRIPTION OF THE EMBODIMENTS

The present invention will be further described in detail below in combination with embodiments. It needs to be pointed out that the following embodiments are intended to facilitate the understanding of the present invention, but do not have any limiting effect on it. The following embodiments of the present invention are only limited examples of the present invention, the scope of which is defined by the claims of the present invention.

### Embodiment 1 Preparation of lateral flow detection device for immunoassay detection provided by the present invention

A lateral flow detection device for immunoassay detection of novel coronavirus prepared in this embodiment is shown in FIGS. 1 and 2, including a test strip, and according to a liquid flow direction, from upstream to downstream, sequentially including a sample area 101, a labeling area 102, a detection area 103 and a water-absorbing area 104. The water-absorbing area is prepared by using general water-absorbing filter paper as a water-absorbing pad; the sample area 101 uses a sample application pad whose material is glass fiber, so that a sample added through a sample adding hole S flows onto the glass fiber, which is then mixed with the sample to flow onto the labeling area 102; the labeling area is made into a labeling pad, including labeling particles (such as gold particles, fluorescent particles, latex particles or dyes, or other colored labeling substances) conjugated antigens or antibodies, then a labeling mixture is sprayed on a polyester film through spraying equipment to make the labeling pad, and a labeling substance on the labeling pad can flow with flow of a liquid; the detection area uses a nitrocellulose membrane, antibodies or antigens on a detection line are dissolved with a buffer solution PBS, and then dot-membrane equipment is used for drawing a line on the nitrocellulose membrane, so that a distance between different antibodies is 3 to 8 millimeters, and then the nitrocellulose membrane is put in an oven to be dried for standby use, the antibodies, antigens or other binding substances processed on the membrane generally do not move, and the detection area includes a detection area 105 and a detection result control area 106.

After preparation of the water-absorbing area 104, the sample area 101, the labeling area 102 and the detection area 103 are respectively completed, they are assembled so that one end of the sample application pad is superimposed on one end of the labeling pad, the other end of the labeling pad is superimposed on the nitrocellulose membrane, and the nitrocellulose membrane at one end of a control line is superimposed by the water-absorbing filter paper, in this way, an entire test strip is formed and then assembled in a test card, wherein the sample adding hole S on the test card corresponds to the sample application pad, the nitrocellulose membrane corresponds to a degree window, and in addition, the sample adding hole S is located downstream of a buffer solution hole B.

When a sandwich method is used for making a test element, it may be a direct method of double-antibody sandwich or an indirect method. For example, if an antigen A1 in a sample needs to be detected, a first antibody AB1 to the antigen A1 coupled to a color label can be processed on a labeling pad, and a second antibody AB2 to the antigen A1 is immobilized on a detection line. If the content of the antigen A1 in the sample is more, labeled substances captured on the detection line are more, and the color is darker, indicating the higher content.

In some another embodiments, when a competition method is used for test, such as when it is desired to detect drugs of abuse in urine or saliva, such as THC (a small drug molecule, hapten - hardly immunogenic), antibodies corresponding to antigens with THC are treated on a labeling pad and coupled to colored particles, and the antigens with the THC or similar substances are treated on a detection line. When a sample contains THC, the THC binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

These test strips can be used for detection of the analyte alone, or can be placed in a test card for detection, for example, there are a window for observing results and a window for liquid dripping in the test card, the window for liquid dripping is generally located over a sample application area, and a window for reading detection results is located over a detection area.

### Embodiment 2: Influence of sampling devices prepared with different sponges on detection results of Δ9-THC

Δ9-THC, also known as tetrahydrocannabinol, Δ9-tetrahydrocannabinol, Δ1-THC (according to the old nomenclature), and dronabinol (a chemically synthesized drug), THC for short, is a main psychoactive substance in cannabis, and is naturally present in resin secreted by stamens of female flowers of Moraceae plant *Cannabis sativa L..* After inhaling cannabis containing this product, people are both excited and inhibited, and after smoking it, thoughts rise and fall, spirit is excited, and people are consciously euphoric; or people are indulged in depression and panic. After long-term use, people are mentally depraved and severely incapacitated for work. It is a strictly controlled substance. However, during development of in-vitro diagnostic reagents of Δ9-THC, it is found that Δ9-THC is very easily adsorbed by a traditional sponge head for sampling, resulting in that it is undetectable and false negative detection results are shown. Higher.

A THC reagent strip: when a competition method is used for test, such as when it is desired to detect drugs of abuse in urine or saliva, such as THC (a small drug molecule, hapten - hardly immunogenic), antibodies corresponding to antigens with THC are treated on a labeling pad and coupled to colored particles, and the antigens with the THC or similar substances are treated on a detection line. When a sample contains THC, the THC binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

### Embodiment 2.1: Influence on plate detection results of Δ9-THC in saliva samples (competition method)-gold particle labeling colorimetric method

In this embodiment, standard sample solutions (prepared with saliva) containing 20, 40, 60 and 80 ng/mL of Δ9-THC are respectively prepared, and two sponge heads of different materials are used for sampling, the first of which is a sampling head prepared from a melamine foam, the second of which is a sponge head (polypropylene sponge pp) prepared from polypropylene, sizes of the sponge heads are the same, and a sampling adsorption capacity is 2 ml. After a sample is physically extruded, 0.12 ml of the sample is taken to be dropped into a sample application area of a detection reagent strip, a content of Δ9-THC in the sample is detected by a competition method, and after 10 minutes, reading and contrastive photographing are carried out. Detection results are divided into G1 to G10 according to a colourimetric card (FIG. 3), wherein being less than or equal to G4 is positive, being higher than G4 is negative, and specific results are shown in Table 1; the higher the reading, the less the content of drugs.

**Table 1. Comparison of adsorption properties of different materials to Δ9-THC**

| Δ9-THC concentration (ng/mL) | Melamine foam | | | Control (polypropylene sponge pp) | | |
|---|---|---|---|---|---|---|
| 0 | G9.5 | G9.5 | G9.5 | G9.5 | G9.5 | G9.5 |
| 20 | G6 | G6 | G6 | G8 | G8 | G8 |
| 40 | G2 | G2 | G2 | G5.5 | G5.5 | G6 |
| 60 | G1 | G1 | G1 | G5 | G5 | G5 |
| 80 | G1 | G1 | G1 | G5 | G4.5 | G5 |

As can be seen from Table 1, compared with a traditional sampling head prepared in control (polypropylene sponge), detection results of samples collected by using the sampling head prepared from the melamine foam have a big difference. When the sample contains Δ9-THC of 20 ng/mL, a detection result of the sampling head prepared from the melamine foam is G6, which is significantly higher than a detection result (G8) in control. When the sample contains Δ9-THC of 40, 60 and 80 ng/mL, a detection result of the sampling head prepared from the melamine foam is G2 or G1, which is lower than G4 and is positive, while a detection result in control is G5, and there is a false negative result. The detection result of the sampling head prepared from the melamine foam is significantly higher than that in control. It can be seen that the sampling head prepared from the melamine foam can significantly improve detection sensitivity of the Δ9-THC in the sample, and the reason is that the melamine foam can significantly reduce adsorption to Δ9-THC during sample collection and restore authenticity of the samples, so that false negatives can be prevented.

### Embodiment 2.2: Influence on plate detection results of Δ9-THC in saliva samples (competition method)-gold-labeled particle color reading method

In addition to color comparison to judge and read test values, a machine is used for more accurate Δ9-THC saliva colloidal gold machine reading of test results (drug trace detector AFS-1000: A202006929.). The greater the T value, the less the drug content in tested samples, and specific results are shown in Table 2.

**Table 2. Test results of detection line read by using equipment (comparison of adsorption properties of different materials to Δ9-THC)**

| Δ9-THC concentration (ng/mL) | Melamine foam T value reading | | | Polypropylene sponge pp T value reading | | |
|---|---|---|---|---|---|---|
| 0 | 611.53 | 556.95 | 613.50 | 621.48 | 570.78 | 604.86 |
| 20 | 160.99 | 171.23 | 189.27 | 348.62 | 345.88 | 369.95 |
| 60 | 21.03 | 20.09 | 22.07 | 136.28 | 104.62 | 136.82 |

Conclusion: when a negative solution is tested, there is no significant difference between control group data and experimental group data; when a positive solution is tested, when samples are all 20 ng/mL, for sampling by using the melamine foam, a T value is 160 to 190, while for sampling by using a traditional polypropylene sponge, a T value reading is 350 to 370, and there is almost twice the difference, indicating that for samples sampled with the melamine foam, adsorption to Δ9-THC is significantly reduced and is close to a real value, while for sample sampling by using the polypropylene sponge, false negative results may be caused due to large adsorption to Δ9-THC.

### Embodiment 2.3: Influence on plate detection results of Δ9-THC in saliva samples (competition method)-fluorescent labeling reading method

When fluorescent labeling is used, similar to gold-labeled particles, only substances for signal producing on a labeling pad change. Therefore, when a competition method is used for test, such as when it is desired to detect drugs of abuse in urine or saliva, such as THC (a small drug molecule, hapten - hardly immunogenic), antibodies corresponding to antigens with THC are treated on a labeling pad and coupled to fluorescent substances, and the antigens with the THC or similar substances are treated on a detection line. When a sample contains THC, the THC binds to fluorescently coupled antibodies, and when it flows to the detection line, the antigens on the detection line and an analyte in the sample competitively bind to the antibodies, so that if the content of small molecules in the sample is more, fluorescent substances captured on the detection line are less, indicating a positive result; on the contrary, if the content of small molecules in the sample is less, color fluorescent substances captured on the detection line are more, indicating a negative result.

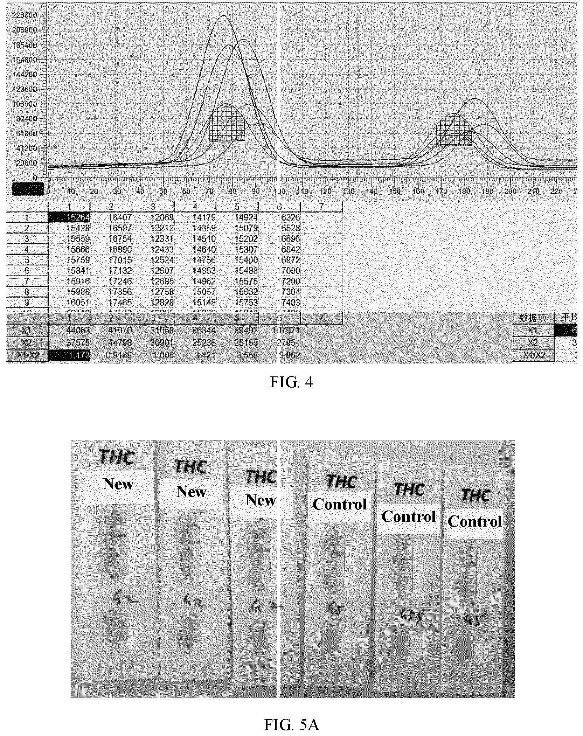

Results: Δ9-THC saliva fluorescent test strip reading. The higher the value of X1/X2 (test line T/test result control line C), the stronger the line, and the lower the drug content in the sample. Each group of data in this experiment is repeated three times, machine-reading results are averaged, and results thereof are shown in Table 3 below (FIG. 4).

**Table 3: Fluorescent test strip reading (X1/X2)**

| Δ9-THC concentration (ng/mL) | Experimental (X1/X2) melamine foam | | | Control (X1/X2) polypropylene sponge pp | | |
|---|---|---|---|---|---|---|
| 0 | 5.133 | 5.429 | 5.605 | 5.656 | 5.357 | 4.973 |
| 20 | 1.173 | 0.9168 | 1.005 | 3.421 | 3.558 | 3.862 |
| 30 | 0.2535 | 0.2651 | 0.2365 | 0.8362 | 1.029 | 0.6698 |

When a negative solution is tested, there is no significant difference between control group data and experimental group data; when a positive solution is tested, when samples are all 20 ng/mL, for sampling by using the melamine foam, an X1/X2 value is 0.9 to 1.173, while for sampling by using a traditional polypropylene sponge, an X1/X2 value reading is 3.4 to 3.8, and there is almost twice the difference, indicating that for samples sampled with the melamine foam, adsorption to Δ9-THC is significantly reduced and is close to a real value, while for sample sampling by using the polypropylene sponge, false negative results may be caused due to large adsorption to Δ9-THC. From FIG. 4, in a concentration of 20 ng/mL, three columns of data on the left are fluorescence reading values of sampling by the melamine foam, and three columns of data on the right are degrees of absorption to the same samples by traditional polypropylene sponge.

Meanwhile, we also have conducted similar experiments on polyvinyl alcohol sponge (PVA) and polyurethane sponge (PU), results thereof are similar to those of polypropylene sponge (PP). Detection results of a sampling head prepared from the melamine foam are significantly higher than those of the polyvinyl alcohol sponge (PVA) and the polyurethane sponge (PU), indicating that an adsorption capacity to THC of a sampler prepared by using the melamine foam is obviously less than that of the polyvinyl alcohol sponge (PVA) (Embodiment 3) and the polyurethane sponge (PU).

### Embodiment 3: Influence on detection results of Δ9-THC in saliva (competition method)

In this embodiment, two sponge heads of different materials are used for saliva sampling, the first of which is a sampling head prepared from a melamine foam, the second of which is a sponge head (polyvinyl alcohol sponge PVA) prepared from cotton, sizes of the sponge heads are the same, and a sampling adsorption capacity is 2 ml. After a sample is physically extruded, 0.5 ml of the sample is taken to be dropped into a detection reagent strip, and a content of Δ9-THC in the sample is detected by a competition method. It is known that standard saliva contains Δ9-THC of 20, 40, 60 and 80 ng/mL respectively, detection results are divided into G1 to G10 according to a colourimetric card (FIG. 3), wherein being less than or equal to G4 is positive, being higher than G4 is negative, and detection results are shown in Table 4.

**Table 4. Comparison of adsorption properties of different materials to Δ9-THC in saliva**

| Δ9-THC concentration (ng/mL) | Melamine foam | | | Control (polyvinyl alcohol sponge PVA) | | |
|---|---|---|---|---|---|---|
| 20 | G6 | G6 | G6 | G8 | G8 | G8 |
| 40 | G1 | G1 | G1 | G6 | G6 | G6 |
| 60 | G1 | G1 | G1 | G5 | G5 | G5 |
| 80 | G1 | G1 | G1 | G4 | G4 | G4 |

As can be seen from Table 2, for saliva samples, compared with a sampling head prepared in control (polyvinyl alcohol sponge PVA), detection results of Δ9-THC of samples collected by using the sampling head prepared from the melamine foam also have a big difference. When saliva contains Δ9-THC of 20 ng/mL, a detection result of the sampling head prepared from the melamine foam is G6, while a detection result in control is G8. When the saliva contains Δ9-THC of 40 to 60 ng/mL, a detection result of the sampling head prepared from the melamine foam is G1, which is lower than G4 and is positive, while a detection result in control is G5 or G6, which is a negative result. The detection result of the sampling head prepared from the melamine foam is significantly higher than that in control. It can be seen that the sampling head prepared from the melamine foam can significantly improve detection sensitivity of the Δ9-THC in the sample, and the reason is that the melamine foam can significantly reduce adsorption to Δ9-THC during saliva sample collection and restore authenticity of the samples, so that false negatives can be prevented.

### Embodiment 4: Influence on detection results of Δ9-THC, AMP, COC, MET and OPI in saliva simultaneously existing in samples (competition method)-color card colorimetric method

A test strip is prepared according to the method in Embodiment 1 above, and only target substances are different. For details, referring to a production method in FIG. 1 as follows (other conditions are the same).

THC: when it is Δ9-THC (a small drug molecule, hapten - hardly immunogenic), antibodies corresponding to antigens with Δ9-THC are treated on a labeling pad and coupled to colored particles, and the antigens with the Δ9-THC or similar substances are treated on a detection line. When a sample contains Δ9-THC, the Δ9-THC binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

AMP: when it is AMP (a small drug molecule, hapten - hardly immunogenic), antibodies corresponding to antigens with AMP are treated on a labeling pad and coupled to colored particles, and the antigens with the AMP or similar substances are treated on a detection line. When a sample contains AMP, the AMP binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

COC: when it is COC (a small drug molecule, hapten - hardly immunogenic), antibodies corresponding to antigens with COC are treated on a labeling pad and coupled to colored particles, and the antigens with the COC or similar substances are treated on a detection line. When a sample contains COC, the COC binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

MET: when it is MET (a small drug molecule, hapten - hardly immunogenic), antibodies corresponding to antigens with MET are treated on a labeling pad and coupled to colored particles, and the antigens with the MET or similar substances are treated on a detection line. When a sample contains MET, the MET binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

OPI: when it is OPI (a small drug molecule, hapten - hardly immunogenic), antibodies corresponding to antigens with OPI are treated on a labeling pad and coupled to colored particles, and the antigens with the OPI or similar substances are treated on a detection line. When a sample contains OPI, the OPI binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

Different contents of standards are prepared by using the same saliva, the first of which is a sampling head prepared from a melamine foam, the second of which is a sponge head (polypropylene sponge pp) prepared from cotton, sizes of the sponge heads are the same, and a sampling adsorption capacity is 2 ml. After a sample is physically extruded, 500 microlitres of the sample is taken to be dropped into a sample application area of a detection reagent strip, and a content of Δ9-THC in the sample is detected by a competition method. It is known that standard saliva contains Δ9-THC, AMP, COC, MET and OPI, corresponding concentrations are shown in Table 5; detection results are divided into G1 to G10 according to a colourimetric card (FIG. 3), wherein being less than or equal to G4 is positive, being higher than G4 is negative, and detection results are shown in Table 5.

When a positive solution is tested, a line color intensity of a THC reagent strip for sampling by a melamine foam is significantly lower than that of polypropylene sponge for sampling. Because it is a competition method, the greater the intensity, the less the content of an analyte in a sample, and the smaller the intensity, the higher the content of an analyte in a sample after sucked by a sampling head and squeezed. From the above experimental results, when THC is mixed with AMP, COC, MET and OPI, it does not affect sensitivity of other detection substances. This also shows that by using the melamine foam for sampling, when the sample contains a variety of drug abuse small molecules, it does not affect normal detection results of THC, and can still reduce adsorption action of THC.

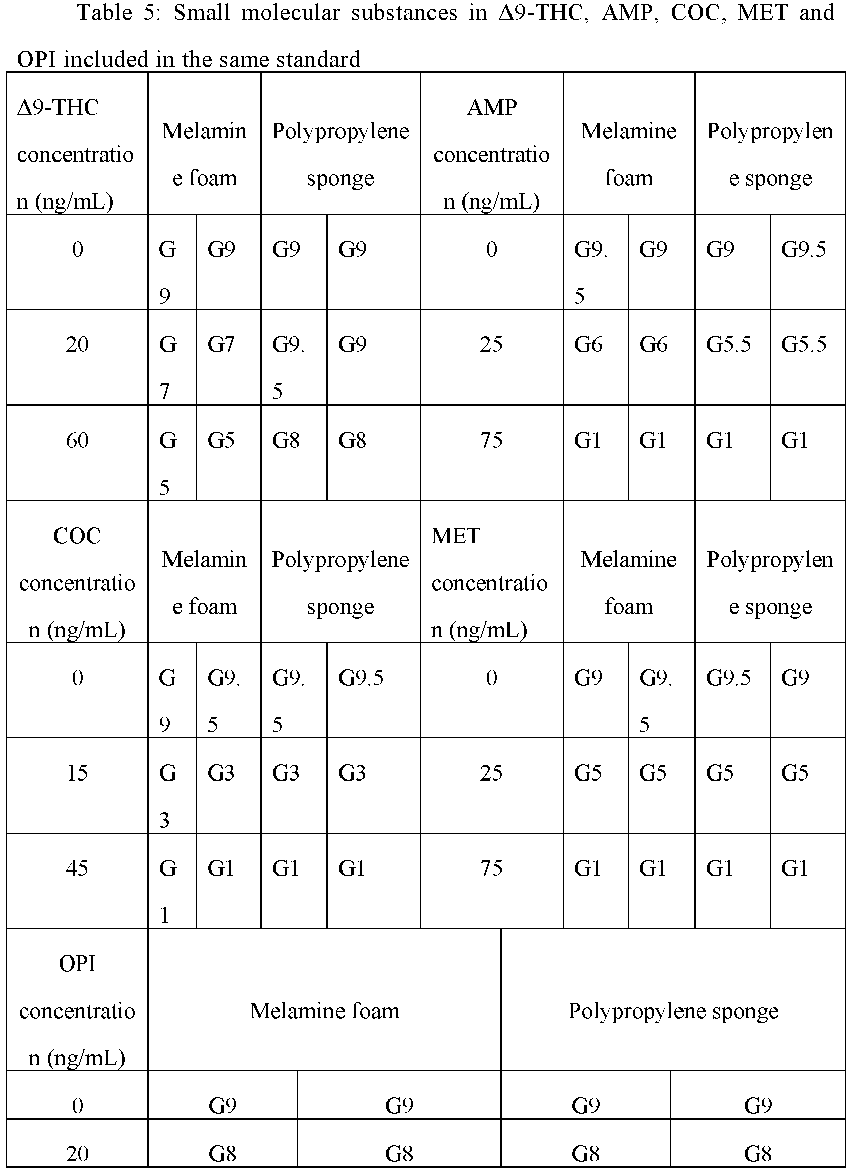

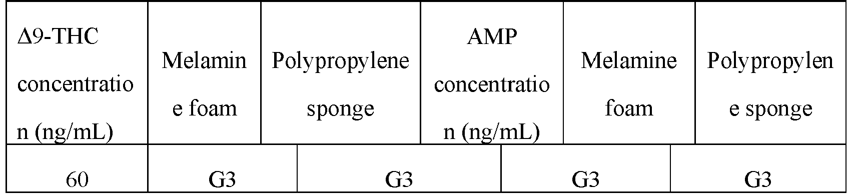

### Embodiment 5.1: Influence on detection results of Δ9-THC-COOH in saliva (competition method)-colorimetric method

In this embodiment, sample solutions (prepared with saliva) containing 20, 40 and 60 ng/mL of Δ9-THC-COOH are respectively prepared, and two sponge heads of different materials are used for sampling, the first of which is a sampling head prepared from a melamine foam, the second of which is a sponge head (polypropylene sponge) prepared from polypropylene, sizes of the sponge heads are the same, and a sampling adsorption capacity is 2 ml. After a sample is physically extruded, 0.12 ml of the sample is taken to be dropped into a detection reagent strip, and after 10 minutes, reading and contrastive photographing are carried out. The higher the reading, the less the drug content, a standard reading is judged to be greater than G4, which is negative, and a reading is less than or equal to G4, which is positive. Results are shown in Table 6.

Preparation of a test strip refers to the method in Embodiment 1, and is specifically described as follows: THC: when it is Δ9-THC-COOH (a small drug molecule, hapten - hardly immunogenic), antibodies corresponding to antigens with Δ9-THC-COOH are treated on a labeling pad and coupled to colored particles, and the antigens with the Δ9-THC-COOH or similar substances are treated on a detection line. When a sample contains Δ9-THC-COOH, the Δ9-THC-COOH binds to antibodies of the colored particles, and when it flows to the detection line, the antigens on the detection line and an analyte in the sample competitively bind to antibodies, so that if the content of small molecules in the sample is more, colored particles captured on the detection line are less, and the color is lighter or weaker, indicating a positive result; on the contrary, if the content of small molecules in the sample is less, colored particles captured on the detection line are more, and the color is darker or stronger, indicating a negative result.

**Table 6. Result values obtained by using different materials for sampling**

| Table 6 | Melamine foam | | | Polypropylene sponge | | |
|---|---|---|---|---|---|---|
| Serial number | 1 | 2 | 3 | 1 | 2 | 3 |
| Concentrations | 0ng/ml | 20ng/ml | 30ng/ml | 0ng/ml | 20ng/ml | 30ng/ml |
| Reading | G9.5/G9. 5 | G3/G3.5/G 3 | G1/G1/G 1 | G9.5/G9. 5 | G6/G6/G 6 | G5/G4.5/G4. 5 |

It can be seen from the above experimental results that when the melamine foam is used for sampling of saliva samples, a reading is 3 under a positive standard of 20 ng/ml, while a reading is 1 under a condition of 30 ng/ml, which can be directly judged to be a positive result (due to using a competition method), on the contrary, when traditional polypropylene is used for sample adsorption, under the condition of 30 ng/ml, a reading is 4.5 to 5, which is still judged to be a negative result. It shows that the polypropylene has a great adsorption effect on Δ9-THC-COOH when used for sample sampling or processing. In actual, in a sample squeezed from an absorption head, an actual content of Δ9-THC-COOH is significantly less than 30 ng/ml.

### Embodiment 5.2: Influence on detection results of Δ9-THC-COOH in saliva (competition method)-gold-labeled color machine automatic reading

A reagent strip prepared by the method in Embodiment 5.1 and samples are the same, test strips are from the same batch, only a way of reading is not naked-eye colorimetry, but direct numerical values are obtained by a method of machine reading. Δ9-THC-COOH saliva colloidal gold machine reading: the larger the T value, the less the drug content. Specific results are shown in Table 7.

**Table 7: Machine-read numerical values.**

| Δ9-THC-COOH concentration (ng/mL) | Melamine foam T value reading | | | Polypropylene T value reading | | |
|---|---|---|---|---|---|---|
| 20 | 59.89 | 82.28 | 71.68 | 162.45 | 155.55 | 158.30 |
| 30 | 27.54 | 39.28 | 40.93 | 91.45 | 114.99 | 141.58 |

Conclusion: when a negative solution is tested, there is no significant difference between control group data and experimental group data; when a positive solution is tested, when samples are all 20 ng/mL, for sampling by using the melamine foam, a T value is 60 to 82, while for sampling by using a traditional polypropylene sponge, a T value reading is 155 to 162, and there is almost two to three times the difference, indicating that for samples sampled with the melamine foam, adsorption to Δ9-THC-COOH is significantly reduced and is close to a real value, while for sample sampling by using the polypropylene sponge, false negative results may be caused due to large adsorption to Δ9-THC-COOH.

### Embodiment 5.3: Influence on plate detection results of Δ9-THC-COOH in saliva samples (competition method)-fluorescent labeling reading method

A reagent strip prepared by the method in Embodiment 2.3 and samples are the same, test strips are from the same batch, only a way of reading is not naked-eye colorimetry, but direct numerical values are obtained by a method of fluorescence reading. Δ9-THC-COOH fluorescent test strip reading: the higher the X1/X2 value, the stronger the line and the lower the drug content in the sample. Each group of data in this experiment is repeated three times, machine-reading results are averaged, and results thereof are shown in Table 8 below.

**Table 8: Fluorescent reading values**

| Δ9-THC-COOH concentration (ng/mL) | Experimental (X1/X2) melamine foam | | | Control (X1/X2) polypropylene | | |
|---|---|---|---|---|---|---|
| 20 | 3.150 | 3.130 | 3.216 | 4.433 | 4.403 | 4.272 |
| 30 | 2.315 | 1.934 | 2.522 | 4.135 | 4.379 | 4.342 |

Conclusion: when a negative solution is tested, there is no significant difference between control group data and experimental group data; when a positive solution is tested, numerical values in a control group are significantly higher than those in an experimental group. The degree of adsorption of Δ9-THC and Δ9-THC-COOH in the control group is greater than that in the experimental group, and the adsorption resistance of sponge heads in the experimental group is better than that of sponge heads in the control group.

### Embodiment 6.1: Influence on plate detection results of Δ9-THC in urine samples (competition method)-colorimetric card

In this embodiment, sample solutions (prepared with urine) containing 20, 40 and 60 ng/mL of Δ9-THC are respectively prepared, and two sponge heads of different materials are used for sampling, the first of which is a sampling head prepared from a melamine foam, the second of which is a sponge head (polypropylene sponge) prepared from polypropylene, sizes of the sponge heads are the same, and a sampling adsorption capacity is 2 ml. After a sample is physically extruded, 0.12 ml of the sample is taken to be dropped into a detection reagent strip, and after 10 minutes, reading and contrastive photographing are carried out. Urine colloidal gold color card reading: the higher the reading, the less the drug content, a standard reading is judged to be greater than G4, which is negative, and a reading is less than or equal to G4, which is positive.

**Table 9. Influence of adsorption of different materials to Δ9-THC in urine**

| **Δ9-THC** | Experimental group (melamine foam) (new) | | | Control group (polypropylene) | | |
|---|---|---|---|---|---|---|
| Serial number | / | 1-2-3 | 4-5-6 | / | 7-8-9 | 10-11-12 |
| Concentrations | 0ng/ml | 20ng/ml | 30ng/ml | 0ng/ml | 20ng/ml | 30ng/ml |
| Reading | G9.5/G9.5 | G2/G2/G2 | G1/G1/G1 | G9.5/G9.5 | G5/G5.5/G5 | G6/G6/G5.5 |

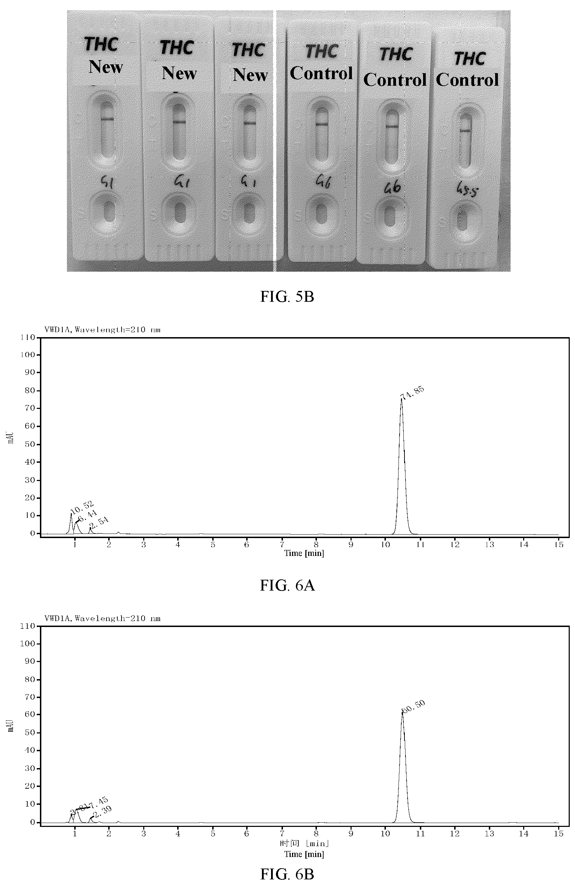

It can be seen from the above experimental results that (FIGS. 5A and 5B), when the melamine foam is used for sampling of urine samples, a reading is 2 under a positive standard of 20 ng/ml, while a reading is 1 under a condition of 30 ng/ml, which can be directly judged to be a positive result (due to using a competition method), on the contrary, when traditional polypropylene is used for sample adsorption, under the condition of 30 ng/ml, a reading is 6, which is still judged to be a negative result. It shows that the polypropylene has a great adsorption effect on Δ9-THC when used for sample sampling or processing. In actual, in a sample squeezed from an absorption head, an actual content of Δ9-THC is less than 30 ng/ml.

### Embodiment 6.2: Influence on plate detection results of Δ9-THC and Δ9-THC-COOH in urine samples (competition method)-fluorescent reading

Δ9-THC/Δ9-THC-COOH concentration urine fluorescence machine reading: the larger the T value, the less the drug content in tested samples.

**Table 10. Influence of adsorption of different materials to Δ9-THC in urine**

| Δ9-THC concentration (ng/mL) | Experimental (X1/X2) (melamine foam) | | | Control (X1/X2) (polypropylene) | | |
|---|---|---|---|---|---|---|
| 0 | 3.836 | 4.099 | 3.696 | 3.944 | 3.581 | 3.695 |
| 30 | 0.4517 | 0.3669 | 0.4705 | 3.285 | 3.225 | 3.518 |

| Δ9-THC-COOH concentration (ng/mL) | Experimental (X1/X2) (melamine foam) | | | Control (X1/X2) (polypropylene) | | |
|---|---|---|---|---|---|---|
| 20 | 0.5909 | 0.7531 | 0.7416 | 3.165 | 3.152 | 3.341 |
| 30 | 0.2448 | 0.1764 | 0.1861 | 1.849 | 2.154 | 2.252 |

Conclusion: when a negative solution is tested, there is no significant difference between control group data and experimental group data; when a positive solution is tested, numerical values in a control group are significantly higher than those in an experimental group. The degree of adsorption of Δ9-THC and Δ9-THC-COOH in the control group is greater than that in the experimental group, and the adsorption resistance of sponge heads in the experimental group is better than that of sponge heads in the control group.

### Embodiment 7.1: Influence on plate detection results of Δ9-THC in blood samples (competition method)-colorimetric card

In this embodiment, sample solutions (prepared with whole blood) containing 20 and 60 ng/mL of Δ9-THC are respectively prepared, and two sponge heads of different materials are used for sampling, the first of which is a sampling head prepared from a melamine foam, the second of which is a sponge head (polypropylene sponge) prepared from polypropylene, sizes of the sponge heads are the same, and a sampling adsorption capacity is 2 ml. After a sample is physically extruded, 80 uL of the sample is taken to be dropped into a detection reagent strip, and after 10 minutes, reading and contrastive photographing are carried out. Urine colloidal gold color card reading: the higher the reading, the less the drug content, a standard reading is judged to be greater than G4, which is negative, and a reading is less than or equal to G4, which is positive.

**Table 11. Influence of adsorption of different materials to Δ9-THC in blood**

| Δ9-THC concentration (ng/mL) | Experimental (melamine foam) | | | Control (polypropylene) | | |
|---|---|---|---|---|---|---|
| 0 | G9.5 | G9.5 | G9.5 | G9.5 | G9.5 | G9.5 |
| 20 | G8 | G8 | G8 | G8.5 | G8.5 | G8.5 |
| 60 | G6 | G6.5 | G6.5 | G8 | G8.5 | G8.5 |

It can be seen from the above experimental results that when the melamine foam is used for sampling of blood samples, a reading is 6.5 under a positive standard of 60 ng/ml, however, when traditional polypropylene is used for sample adsorption, a reading is 8.5. A concentration of blood samples sampled by the melamine foam is higher than that of blood samples sampled by polypropylene, so the advantage of low adsorption of using the melamine foam as a sampling device in collection of blood samples still exists.

### Embodiment 8: Comparison of HPLC concentrations of Δ9-THC samples before and after adsorption by new and old sponges

1. Instruments and conditions: Agilent high performance liquid chromatograph HPLC-1260; chromatographic conditions: C18 reversed-phase chromatographic column, a wavelength of 210 nm, mobile phases: high-purity water and acetonitrile, an acetonitrile ratio of 70%; a flow rate of 1 ml/min, a column temperature of 40°C, and an injection volume of 10 µl.
2. Standard solutions of 5 ng/ml, 25 ng/ml, 50 ng/ml, 75 ng/ml and 100 ng/ml of tetrahydrocannabinol are prepared to make a calibration curve of concentrations and peak areas: y=0.116x+0.002, r=0.99996, R2=0.99992.
3. 500 µl of a standard solution of 100 ng/ml of tetrahydrocannabinol Δ9-THC is taken (an injection volume of 10 µl); two sponge heads of different materials are used for sampling, the first of which is a sampling head prepared from a melamine foam, the second of which is a sponge head (polypropylene sponge) prepared from polypropylene, sizes of the sponge heads are the same, and a sampling adsorption capacity is 500µl. After samples are physically extruded, 10 µl of injection volumes are taken for each to obtain chromatograms 6A, 6B and 6C and Table 12.

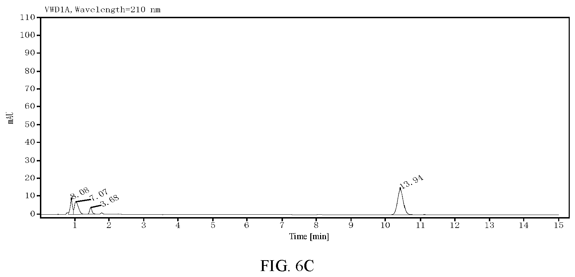

It can be seen from FIGS. 6A to 6C and Table 12 that peak appearance time of three different solutions is 10 to 11 minutes. For a standard (without treatment absorption by any material), a peak height thereof is 74.845 and an area thereof is 868.6890, and a concentration value obtained by a standard formula is 100.770. The reason for more than 100 ng/ml is an error caused in a preparation process. After a standard of the same concentration is absorbed by the melamine foam, a squeezed sample is injected by HPLC, a peak height thereof is 60.500, an area thereof is 706.3172, and a concentration value obtained by the standard formula is 81.935. This shows that after absorption by the melamine foam, 20% of a target substance is lost relative to an unabsorbed sample, that is to say, about 20% of Δ9-THC is absorbed, but 80% of Δ9-THC is retained in the squeezed sample. For a solution after adsorption by the polypropylene sponge, after injection by HPLC, a peak height thereof is 13.939, an area thereof is 161.1271, and a concentration value obtained by the standard formula is 18.693. This shows that after adsorption by the traditional polypropylene sponge, about 80% of Δ9-THC is adsorbed, while only about 20% of Δ9-THC is remained in the squeezed sample. Therefore, it is indicated that by absorbing and squeezing a sample by the newly discovered melamine foam of the present invention, adsorption of Δ9-THC can be significantly reduced and detection sensitivity is improved. It needs to be noted that peak heights in accompanying drawings are all accurate to 2 decimal places, while those in the table are actual peak heights.

**Table 12: Responses after different materials absorb standards**

| **Names** | **Retention time [min]** | **Peak area** | **Peak height** | **Content (ng/ml)** |
|---|---|---|---|---|
| FIG. 6A | 10.451 | 868.6890 | 74.845 | 100.770 |
| FIG. 6B | 10.478 | 706.3172 | 60.500 | 81.935 |
| FIG. 6C | 10.415 | 161.1271 | 13.939 | 18.693 |

### Embodiment 9: Compositional analysis spectra of new/old sponges

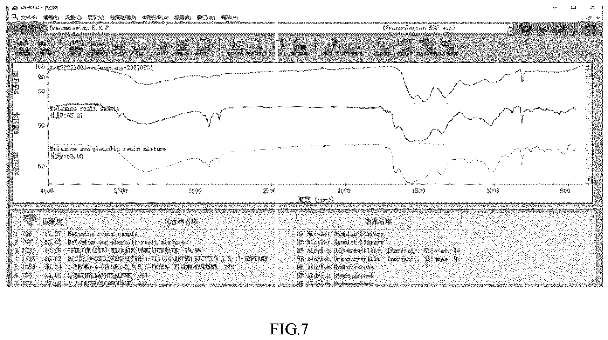

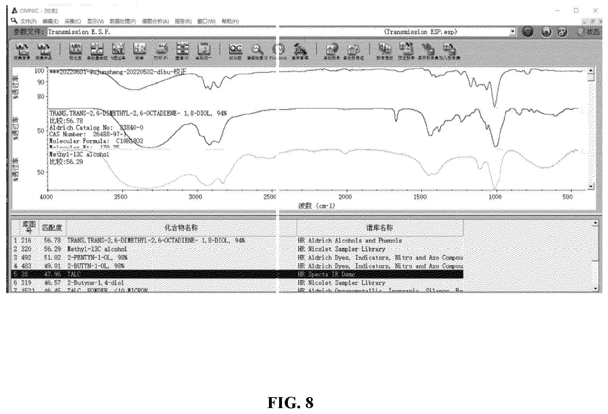

In order to analyze specific chemical compositions of a melamine foam from chemical compositions, we carry out infrared analysis on the melamine foam and commonly used polypropylene synthetic resin. Specific results are as follows, see infrared spectra and FIGS. 7 and 8 for details. It can be seen from the above infrared spectra that wavenumbers of the melamine foam and a polypropylene sponge are 1000 to 1500 cm⁻¹, and their infrared absorption is very different, indicating that functional groups of main compositions of the two sponges are different. Further comparison of a database shows that a main composition of the melamine foam is melamine resin or melamine and phenolic resin, which is a copolycondensation product of melamine, phenol and formaldehyde. A sponge in FIG. 2 is polypropylene synthetic resin.

### Embodiment 10 Control experiments in novel coronavirus antigen detection

A preparation method of a novel coronavirus antigen test paper used in this experiment is the same as that in Embodiment 1. This experiment adopts a sandwich method. The larger the T-line reading, the higher the protein content in samples. Melamine foams and conventional sponges of the same size and shape are respectively used for collecting 50 ul of novel coronavirus N protein samples (standards), then the samples are respectively added to 500 ul of a lysis solution for extraction for 15 seconds, and 100 ul of them are dropped into a detection test paper board, and after 10 minutes, reading is carried out.

**Table 13: Comparison of novel coronavirus antigen detection results**

| N protein concentrations | Not adsorbed by sponges | | Adsorbed by melamine foams | | Adsorbed by old sponges (non-melamine sponges) | |
|---|---|---|---|---|---|---|
| 150pg/ml | G6 | G6 | G4 | G4 | G4 | G4 |
| 450pg/ml | G7 | G7 | G5 | G5 | G4 | G4 |

Conclusion: in samples with a sample concentration of 450 pg/ml, a reading without sponge adsorption is G7, a reading after adsorption by the melamine foam is G5, and a reading after adsorption by the old sponge is G4. It can be seen that a reading of sampling by the melamine foam is closer to a true reading.

All patents and publications mentioned in the specification of the present invention indicate that these are disclosed techniques in the art, and the present invention can use them. All patents and publications cited herein are also listed in references as each publication is specifically and individually referred and cited. The present invention described herein may be implemented in the absence of any element or elements, limitation or limitations, no such limitation specifically stated herein. For example, the terms "comprising", "essentially consisting of ..." and "consisting of ..." in each embodiment herein may be replaced by either of the remaining two terms. So-called "one" here only means "one", and it does not exclude that only one is included, and it may also mean that two or more are included. The terms and expressions used herein are description modes, and are not limited by them. There is no any intention here to indicate that these terms and interpretations described in this specification exclude any equivalent features, but it can be known that any suitable changes or amendments may be made within the scope of the present invention as defined by the appended claims.

## Claims

1. A device for processing a liquid sample comprising a melamine material for absorbing the liquid sample and a test element, the device being configured such that when a part of the liquid sample is released from the melamine material, tetrahydrocannabinol (THC) in the released part of the liquid sample is detected by the test element to determine whether drug abuse small molecules exist in the liquid sample.

2. The device according to claim 1, wherein the melamine material comprises a melamine porous material or a melamine porous water-absorbing material.

3. The device according to claim 2, wherein the melamine porous material comprises a melamine foam.

4. The device according to one of claims 1-3, wherein the melamine foam is of a planar film-like structure or a three-dimensional block-like structure.

5. The device according to one of claims 1-4, wherein the planar film-like structure is a filter membrane in any of circle, square, ellipse and polygon; and the block-like structure is a cylinder, a cuboid, a cube, a cone, or any other three-dimensional body.

6. The device according to one of claims 1-5, wherein the liquid sample is any one of saliva, urine, blood, sputum, lymph, plasma, semen, lung aspirates, and a cerebrospinal fluid.

7. The device according to claim 1, wherein the tetrahydrocannabinol comprises Δ9-THC or Δ9-THC-COOH.

8. The device according to claim 1, further comprising a rod whose end is covered with a hydrophilic fibrous filler, so as to absorb the liquid sample, wherein the fibrous filler is sequentially deposited at the end in an electrostatic field according to a certain direction to form a brush-shaped layer to cover the end of the rod, the fibrous filler is perpendicular to a surface of the rod, and fibers are melamine fibers.

9. The device according to claim 8, wherein the brush-shaped layer has a same thickness to absorb the liquid sample by a capillary effect.

10. The device according to claim 8, wherein a thickness of the fibrous filler is in a range of 0.5 to 7 mm, and a weight of the fibrous filler is in a range of 1.0 to 22.0 dtex.

11. The device according to claim 8, wherein a geometry of the end is a circle.

12. The device according to claim 11, wherein a geometry of the end is a pointed circular arch.

13. A method of processing a liquid sample, using the device of any of the previous claims, **characterized by** comprising the following steps:
contacting the liquid sample with an absorbing material, wherein the absorbing material comprises a melamine material;
releasing a part of the liquid sample from the absorbing material after the absorbing material absorbs the liquid sample;
detecting tetrahydrocannabinol (THC) in the released part of the liquid sample to determine whether drug abuse small molecules exist in the liquid sample.

14. The method according to claim 13, wherein releasing the part of the liquid sample from the absorbing material comprises squeezing the absorbing material or eluting the absorbing material with a treatment liquid to dissolve the liquid sample.

15. The method according to claim 13, wherein the melamine material is a melamine porous material or a melamine porous water-absorbing material.

## Patentansprüche

1. Vorrichtung zum Verarbeiten einer flüssigen Probe, umfassend ein Melaminmaterial zum Absorbieren der flüssigen Probe und ein Testelement, wobei die Vorrichtung derart konfiguriert ist, dass, wenn ein Teil der flüssigen Probe aus dem Melaminmaterial freigesetzt wird, Tetrahydrocannabinol (THC) in dem freigesetzten Teil der flüssigen Probe durch das Testelement nachgewiesen wird, um zu bestimmen, ob kleine Drogenmissbrauchsmoleküle in der flüssigen Probe vorhanden sind.

2. Vorrichtung gemäß Anspruch 1, wobei das Melaminmaterial ein poröses Melaminmaterial oder ein poröses wasserabsorbierendes Melaminmaterial umfasst.

3. Vorrichtung gemäß Anspruch 2, wobei das poröse Melaminmaterial einen Melaminschaum umfasst.

4. Vorrichtung gemäß einem der Ansprüche 1 bis 3, wobei der Melaminschaum eine flächige folienartige Struktur oder eine dreidimensionale blockartige Struktur hat.

5. Vorrichtung gemäß einem der Ansprüche 1 bis 4, wobei die flächige folienartige Struktur eine Filtermembran in einem beliebigen von Kreis, Quadrat, Ellipse und Polygon ist; und die blockartige Struktur ein Zylinder, ein Quader, ein Würfel, ein Kegel oder ein beliebiger anderer dreidimensionaler Körper ist.

6. Vorrichtung gemäß einem der Ansprüche 1 bis 5, wobei die flüssige Probe ein beliebiges von Speichel, Urin, Blut, Sputum, Lymphe, Plasma, Sperma, Lungenaspiraten und einem Zerebrospinalfluid ist.

7. Vorrichtung gemäß Anspruch 1, wobei das Tetrahydrocannabinol Δ9-THC oder Δ9-THC-COOH umfasst.

8. Vorrichtung gemäß Anspruch 1, ferner umfassend einen Stab, dessen Ende mit einem hydrophilen faserigen Füllstoff bedeckt ist, um die flüssige Probe zu absorbieren, wobei der faserige Füllstoff am Ende in einem elektrostatischen Feld gemäß einer bestimmten Richtung nacheinander abgeschieden wird, um eine bürstenförmige Schicht zu bilden, um das Ende des Stabes zu bedecken, der faserige Füllstoff senkrecht zu einer Oberfläche des Stabes ist und Fasern Melaminfasern sind.

9. Vorrichtung gemäß Anspruch 8, wobei die bürstenförmige Schicht eine gleiche Dicke aufweist, um die flüssige Probe durch einen Kapillareffekt zu absorbieren.

10. Vorrichtung gemäß Anspruch 8, wobei eine Dicke des faserigen Füllstoffs in einem Bereich von 0,5 bis 7 mm liegt und ein Gewicht des faserigen Füllstoffs in einem Bereich von 1,0 bis 22,0 dtex liegt.

11. Vorrichtung gemäß Anspruch 8, wobei eine Geometrie des Endes ein Kreis ist.

12. Vorrichtung gemäß Anspruch 11, wobei eine Geometrie des Endes ein spitzer Kreisbogen ist.

13. Verfahren zum Verarbeiten einer flüssigen Probe unter Verwendung der Vorrichtung nach einem beliebigen der vorhergehenden Ansprüche, **dadurch gekennzeichnet, dass** es die folgenden Schritte umfasst:
Inkontaktbringen der flüssigen Probe mit einem absorbierenden Material, wobei das absorbierende Material ein Melaminmaterial umfasst;
Freisetzen eines Teils der flüssigen Probe aus dem absorbierenden Material, nachdem das absorbierende Material die flüssige Probe absorbiert hat;
Nachweisen von Tetrahydrocannabinol (THC) in dem freigesetzten Teil der flüssigen Probe, um zu bestimmen, ob kleine Drogenmissbrauchsmoleküle in der flüssigen Probe vorhanden sind.

14. Verfahren gemäß Anspruch 13, wobei das Freisetzen des Teils der flüssigen Probe aus dem absorbierenden Material das Auspressen des absorbierenden Materials oder das Eluieren des absorbierenden Materials mit einer Behandlungsflüssigkeit zum Auflösen der flüssigen Probe umfasst.

15. Verfahren gemäß Anspruch 13, wobei das Melaminmaterial ein poröses Melaminmaterial oder ein poröses wasserabsorbierendes Melaminmaterial ist.

## Revendications

1. Dispositif destiné à traiter un échantillon liquide comprenant un matériau à base de mélamine pour absorber l'échantillon liquide et un élément d'essai, le dispositif étant configuré de sorte que lorsqu'une partie de l'échantillon liquide est libérée du matériau à base de mélamine, le tétrahydrocannabinol (THC) dans la partie libérée de l'échantillon liquide soit détecté par l'élément d'essai pour déterminer si des petites molécules d'abus de drogues existent dans l'échantillon liquide.

2. Dispositif selon la revendication 1, ledit matériau à base de mélamine comprenant un matériau poreux à base de mélamine ou un matériau absorbant l'eau poreux à base de mélamine.

3. Dispositif selon la revendication 2, ledit matériau poreux à base de mélamine comprenant une mousse de mélamine.

4. Dispositif selon l'une des revendications 1 à 3, ladite mousse de mélamine possédant une structure de type film plane ou une structure de type bloc tridimensionnelle.

5. Dispositif selon l'une des revendications 1 à 4, ladite structure de type film plane étant une membrane filtrante dans l'un quelconque d'un cercle, d'un carré, d'une ellipse et d'un polygone ; et ladite structure de type bloc étant un cylindre, un cuboïde, un cube, un cône ou tout autre corps tridimensionnel.

6. Dispositif selon l'une des revendications 1 à 5, ledit échantillon liquide étant l'un quelconque de la salive, de l'urine, du sang, des expectorations, de la lymphe, du plasma, du sperme, des aspirats pulmonaires et d'un liquide céphalorachidien.

7. Dispositif selon la revendication 1, ledit tétrahydrocannabinol comprenant Δ9-THC ou Δ9-THC-COOH.

8. Dispositif selon la revendication 1, comprenant en outre une tige dont l'extrémité est recouverte d'une charge fibreuse hydrophile, de façon à absorber l'échantillon liquide, ladite charge fibreuse étant déposée séquentiellement au niveau de l'extrémité dans un champ électrostatique selon une certaine direction pour former une couche en forme de brosse afin de recouvrir l'extrémité de la tige, ladite charge fibreuse étant perpendiculaire à une surface de la tige, et lesdites fibres étant des fibres de mélamine.

9. Dispositif selon la revendication 8, ladite couche en forme de brosse possédant une même épaisseur pour absorber l'échantillon liquide par un effet capillaire.

10. Dispositif selon la revendication 8, une épaisseur de la charge fibreuse étant comprise dans une plage de 0,5 à 7 mm, et un poids de la charge fibreuse étant compris dans une plage de 1,0 à 22,0 dtex.

11. Dispositif selon la revendication 8, une géométrie de l'extrémité étant un cercle.

12. Dispositif selon la revendication 11, une géométrie de l'extrémité étant une arche circulaire pointue.

13. Procédé de traitement d'un échantillon liquide, à l'aide du dispositif de l'une quelconque des revendications précédentes, caractérisé en comprenant les étapes suivantes :
la mise en contact de l'échantillon liquide avec un matériau absorbant, ledit matériau absorbant comprenant un matériau à base de mélamine ;
la libération d'une partie de l'échantillon liquide du matériau absorbant après que le matériau absorbant a absorbé l'échantillon liquide ;
la détection du tétrahydrocannabinol (THC) dans la partie libérée de l'échantillon liquide pour déterminer si des petites molécules d'abus de drogues existent dans l'échantillon liquide.

14. Procédé selon la revendication 13, ladite libération de la partie de l'échantillon liquide du matériau absorbant comprenant la compression du matériau absorbant ou l'élution du matériau absorbant avec un liquide de traitement pour dissoudre l'échantillon liquide.

15. Procédé selon la revendication 13, ledit matériau à base de mélamine étant un matériau poreux à base de mélamine ou un matériau absorbant l'eau poreux à base de mélamine.
